(19) Europäisches Patentamt
European Patent Office
Office européen des brevets

(11) Veröffentlichungsnummer : **0 090 173**
**B1**

(12) # EUROPÄISCHE PATENTSCHRIFT

(45) Veröffentlichungstag der Patentschrift : **20.08.86**

(51) Int. Cl.[4] : **C 07 D213/73**, C 07 D213/80, C 07 D215/38

(21) Anmeldenummer : **83101710.8**

(22) Anmeldetag : **22.02.83**

(54) **Verfahren zur Herstellung von 2-Aminopyridinen aus 2-Pyridincarbonsäure-N-oxiden.**

(30) Priorität : **31.03.82 CH 1979/82**

(43) Veröffentlichungstag der Anmeldung : **05.10.83 Patentblatt 83/40**

(45) Bekanntmachung des Hinweises auf die Patenterteilung : **20.08.86 Patentblatt 86/34**

(84) Benannte Vertragsstaaten : **AT BE CH DE FR GB IT LI NL SE**

(56) Entgegenhaltungen :
**GB-A- 736 344**
**CHEMISTRY OF HETEROCYCLIC COMPOUNDS, Band 14, Ergänzungsband Teil 3, 1974, Seiten 299-305, Wiley, New York, US R.A. ABRAMOVITCH: "Pyridine and its Derivatives"**

(73) Patentinhaber : **LONZA AG Gampel/Wallis (CH)**

(72) Erfinder : **Quarroz, Daniel, Dr. Balfrinstrasse 21 Visp (Kanton Wallis) (CH)**

(74) Vertreter : **Weinhold, Peter, Dr. et al Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8 D-8000 München 40 (DE)**

**EP 0 090 173 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 2-Aminopyridinen, insbesondere 2-Aminopyridincarbonsäuren, und von 2-Aminochinolin.

Es ist bekannt, 2-Aminopyridine durch Umsatz von Pyridinen mit Natriumamid bei erhöhter Temperatur herzustellen. Diese Synthese ist als « Tschitschibabin-Reaktion » bekannt (Ullmann Enzykl. der techn. Chemie, 4. Aufl., Bd.19, S.602). Der Umgang mit Natriumamid ist unangenehm, da es sich um eine haut- und schleimhautreizende Substanz handelt. Ferner ist bekannt (Beilstein, Bd.22, S.542), dass sich 2-Chlornicotinsäure durch Umsatz mit $NH_3$ bei 170 °C in 2-Aminonicotinsäure umsetzen lässt. Die Ausbeute beträgt etwa 50 %. Ziel der vorliegenden Erfindung ist es, 2-Aminopyridincarbonsäuren und weitere 2-Aminopyridine sowie 2-Aminochinolin nach einem einfachen Verfahren herzustellen.

Erfindungsgemäss wird dies dadurch erreicht, dass man Pyridin-2-carbonsäure-N-Oxide der Formel gemäss Anspruch 1 bzw. Chinaldinsäure-N-Oxid mit niederen aliphatischen Carbonsäureanhydriden und einem tertiären Amin in Gegenwart von Carbonsäurenitrilen umsetzt und anschliessend das gebildete 2-Acylamidopyridin bzw. -chinolin verseift.

Unter niederen aliphatischen Carbonsäureanhydriden sollen solche mit bis zu 6, insbesondere bis zu 5 Kohlenstoffatomen verstanden werden.

Als niedere aliphatische Anhydride werden insbesondere Essigsäureanhydrid, Propionsäureanhydrid und Pivalinsäureanhydrid, vorzugsweise Essigsäureanhydrid, eingesetzt.

Das Carbonsäureanhydrid wird, bezogen auf das eingesetzte N-Oxid, vorzugsweise in 1- bis 10-fachem, insbesondere 1- bis 5-fachem molarem Ueberschuss vorgelegt und kann auch als Lösungsmittel dienen.

Als bevorzugte Carbonsäurenitrile kommen sowohl gesättigte oder ungesättigte aliphatische Nitrile, zweckmässig mit 1 bis 8 C-Atomen, als auch aromatische Nitrile zur Anwendug. Beispielsweise kommen Acetonitril, Acrylonitril, Caprylonitril, Glutaronitril, Methylglutaronitril oder Benzonitril zur Anwendung. Vorzugsweise wird jedoch Acetonitril angewendet.

Bezogen auf das eingesetzte N-Oxid wird vorteilhaft die 15-fache bis 80-fache molare Menge Carbonsäurenitril angewendet.

Als tertiäre Amine können neben Triäthylamin auch andere aliphatische oder aliphatisch/aromatische tertiäre Amine, zum Beispiel Tributylamin, 2,6-Lutidin oder Pyridin verwendet werden. Vorzugsweise wird Triäthylamin eingesetzt. Vorzugsweise kommt ein Verhältnis tertiäres Amin zu N-Oxid zwischen 20 zu 1 und 1 zu 1, insbesondere zwischen 5 zu 1 und 2 zu 1, zur Verwendung.

Als Ausgangsprodukte kommen neben dem Chinaldinsäure-N-Oxid und dem Pyridin-2-carbonsäure-N-oxid auch Pyridindicarbonsäure-N-oxide wie

Pyridin-2,3-dicarbonsäure-N-oxid (Chinolinsäure)
Pyridin-2,4-dicarbonsäure-N-oxid (Lutidinsäure)
Pyridin-2,5-dicarbonsäure-N-oxid (Isocinchomeronsäure)
Pyridin-2,6-dicarbonsäure-N-oxid (Dipicolinsäure),
Pyridintricarbonsäure-N-oxide wie
Pyridin-2,3,4-tricarbonsäure-N-oxid (α-Carbocinchomeronsäure)
Pyridin-2,4,5-tricarbonsäure-N-oxid (Berberonsäure)
Pyridin-2,4,6-tricarbonsäure-N-oxid (Trimesitinsäure)
und Pyridinpentacarbonsäure-N-oxid in Frage.

Die angeführten Pyridincarbonsäure-N-oxide können, soweit noch Positionen verfügbar sind, weiterhin mit Alkylgruppen der Kettenlänge $C_1$ bis $C_8$, insbesondere $C_1$ bis $C_4$, oder mit Arylgruppen kernsubstituiert sein.

Die Umsetzungsreaktion wird vorzugsweise bei einer Temperatur von 0 bis 90 °C, insbesondere bei 20 bis 80 °C, durchgeführt.

Nach diesem Verfahren erhält man überraschenderweise 2-Aminopyridinverbindungen in hohen Ausbeuten, wobei die Reaktion bereits bei relativ tiefen Temperaturen mit grosser Geschwindigkeit abläuft.

Beispiel 1

Herstellung von 2-Amino-5-pyridincarbonsäure aus Isocinchomeronsäure-N-oxid (ICSO).

13 g Essigsäureanhydrid (0,15 Mol), 30 g Triäthylamin (0,3 Mol) und 100 g $CH_3CN$ (2,43 Mol) wurden in einem Kolben vorgelegt und 18,3 g ICSO (0,1 Mol) bei 40 °C portionsweise so zudosiert, dass sich $CO_2$ lebhaft entwickelte. Nach beendeter Zugabe liess man noch ca. 1 Stunde bei 40 °C nachreagieren, bis kein $CO_2$ mehr entwich. Die resultierende braunschwarze Lösung wurde am Rotavapor (30 Torr, 60 °C) eingeengt und der dickflüssige Rückstand durch Zusatz von 10 %iger KOH (End-pH etwa 12), bei 80 °C, 2 Stunden verseift.

Zur Entfernung des Triäthylamins wurde das flüssige Reaktionsgemisch abdestilliert und anschliessend mit konzentrierter HCl angesäuert (pH 5,6). Der hierbei angefallene Niederschlag wurde abgenutscht, mit $H_2O$ gewaschen und bei 45 °C, 20 Torr, getrocknet.

Ausbeute : 6,8 g (titrimerisch bestimmter Gehalt 99,5 %), entsprechend 49 % der Theorie an 2-Amino-5-pyridincarbonsäure. Die Mutterlauge wurde bis pH 1,5 angesäuert. Es fielen noch 1,1 g 6-Hydroxynicotinsäure (Gehalt 95 %) aus. Die Ausbeute betrug 8 %, bezogen auf ICSO.

(Siehe Tabelle Seite 4 f.)

| Beispiel | Edukt 1 Mol | Anhydrid Mol | Amin Mol | Nitril | Temp. °C | Ausbeute % |
|---|---|---|---|---|---|---|
| 2 | ICSO | 1,5 Essigsäureanhyd. | 3 $Et_3N$ | 20 $CH_3CN$ | 0 - 5 | 45,5 ANS |
| 3 | ICSO | 1,5 " | 3 $Et_3N$ | 73 $CH_3CN$ | 40 | 56,5 ANS |
| 4 | ICSO | 5,0 " | 3 $Et_3N$ | 24 $CH_3CN$ | 40 | 45,0 ANS |
| 5 | ICSO | 1,5 " | 7 $Et_3N$ | 24 $CH_3CN$ | 40 | 56 ANS |
| 6 | ICSO | 1,5 " | 3 $Et_3N$ | 24 $CH_3CN$ | 60 - 65 | 53 ANS |
| 7 | ICSO | 1,5 " | 3 $Et_3N$ | 24 $CH_3CN$ | 75 - 80 | 48,5 ANS |
| 8 | ICSO | 1,5 " | 3 $Et_3N$ | 24 CaPCN | 40 | 14 ANS |
| 9 | ICSO | 1,5 " | 3 $Et_3N$ | 20 MGN | 40 | 32 ANS |
| 10 | ICSO | 1,5 " | 3 $Et_3N$ | 38 $CH_2$=CHCN | 40 | 44 ANS |
| 11 | ICSO | 1,5 " | 3 $Et_3N$ | 23 Benzonitril | 40 | 24,5 ANS |
| 12 | ICSO | 1,5 " | 3 $Bu_3N$ | 24 $CH_3CN$ | 40 | 45 ANS |
| 13 | ICSO | 1,5 " | 3 2,6-Lutidin | 24 $CH_3CN$ | 40 | 33 ANS |
| 14 | ICSO | 2 Pivalinsäureanhyd. | 3 $Et_3N$ | 24 $CH_3CN$ | 40 | 39 ANS |
| 15 | PCO | 1,5 Essigsäureanhyd. | 3 $Et_3N$ | 24 $CH_3CN$ | 40 - 45 | 29 2-Amino-pyridin |
| 16 | MPCO | 1,5 " | 3 $Et_3N$ | 24 $CH_3CN$ | 40 - 45 | 18 2-Amino-6-methyl-pyridin (1) |
| 17 | CSO | 1,5 " | 3 $Et_3N$ | 24 $CH_3CN$ | 40 - 45 | 24 APS-3 |
| 18 | LSO | 1,5 " | 3 $Et_3N$ | 24 $CH_3CN$ | 40 - 45 | 50 APS-4 |
| 19 | CCSO | 1,5 " | 3 $Et_3N$ | 24 $CH_3CN$ | 40 - 45 | 34 AC (1) |

ICSO — Isocinchomeronsäure-N-oxid
PCO — Picolinsäure-N-oxid
MPCO — 6-Methylpicolinsäure-N-oxid
CSO — Chinolinsäure-N-oxid
LSO — Lutidinsäure-N-oxid
CCSO — 2-Chinolincarbonsäure-N-oxid oder Chinaldinsäure-N-oxid
$Et_3N$ = Triäthylamin
$Bu_3N$ = Tributylamin
CaPCN = Caprylonitril
MGN = Methylglutaronitril
ANS = 2-Aminopyridincarbonsäure-5
APS-3 = 2-Aminopyridincarbonsäure-3
APS-4 = 2-Aminopyridincarbonsäure-4
AC = 2-Aminochinolin

In der vorstehenden Tabelle bedeutet (1), dass das Endprodukt durch Extraktion im basischen Milieu gewonnen wurde. Als Nebenprodukt bei der Synthese der Aminopyridinverbindungen fallen zum Teil beträchtliche Mengen des entsprechenden Hydroxyprodukts an. So fallen im Beispiel 15 40 % 2-Hydroxypyridin, im Beispiel 17 22 % 2-Hydroxypyridincarbonsäure-3 usw. an.

Die Summe der Ausbeute an Amino- und Hydroxypyridinverbindungen liegen in der Grössenordnung von 60 bis 75 %.

## Patentansprüche

1. Verfahren zur Herstellung von 2-Aminopyridinen der allgemeinen Formel

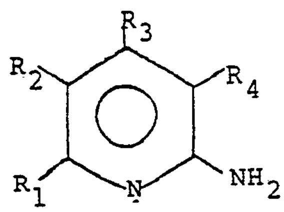

sowie von 2-Aminochinolin aus Pyridin-2-carbonsäure-N-oxiden der allgemeinen Formel

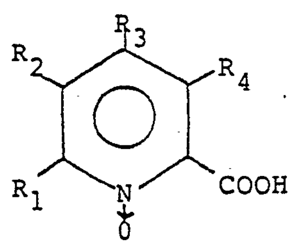

wobei $R_1$, $R_2$, $R_3$ und $R_4$ gleich oder verschieden sind und H, eine —COOH-Gruppe, eine Alkylgruppe mit $C_1$ bis $C_8$ oder eine Arylgruppe bedeuten, bzw. aus Chinaldinsäure-N-oxid, dadurch gekennzeichnet, dass man die genannten N-Oxide mit Anhydriden aliphatischer Carbonsäuren mit bis zu 6 C-Atomen und einem tertiären Amin in Gegenwart von Carbonsäurenitrilen umsetzt und das Umsetzungsprodukt hydrolysiert.

2. Verfahren gemäss Patentanspruch 1, dadurch gekennzeichnet, dass man als Carbonsäureanhydrid Essigsäureanhydrid verwendet.

3. Verfahren gemäss Patentanprüchen 1 und 2, dadurch gekennzeichnet, dass als tertiäres Amin Triäthylamin verwendet wird.

4. Verfahren gemäss Patentanspruch 1 bis 3, dadurch gekennzeichnet, dass man als Carbonsäurenitril aliphatische Carbonsäurenitrile mit 1 bis 8 C-Atomen im Carbonsäurerest oder aromatische Carbonsäurenitrile verwendet.

5. Verfahren gemäss Patentansprüchen 1 bis 4, dadurch gekennzeichnet, dass man pro Mol N-Oxid 1 bis 5 Mol Anhydrid, 2 bis 5 Mol tertiäres Amin und 15 bis 80 Mol Nitril verwendet.

6. Verfahren gemäss Patentansprüchen 1 bis 5, dadurch gekennzeichnet, dass man die Reaktion bei einer Temperatur von 0 bis 90 °C, vorzugsweise bei 20 bis 80 °C, durchführt.

## Claims

1. Process for the production of 2-aminopyridines of the general formula

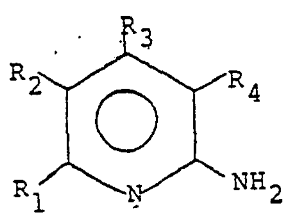

as well as of 2-aminoquinoline from pyridine-2-carboxylic acid N-oxides of the general formula

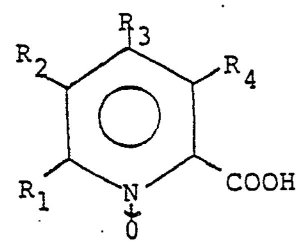

wherein $R_1$, $R_2$, $R_3$ and $R_4$ are the same or different being H, a —COOH-group, an alkyl group with $C_1$ to $C_8$ or an aryl group, and from quinaldic acid N-oxide respectively, characterized in that said N-oxides are reacted with anhydrides of aliphatic carboxylic acids having up to 6 carbon atoms and a tertiary amine in the presence of carboxylic acid nitriles and the reaction product is hydrolyzed.

2. Process according to patent claim 1, characterized in that as carboxylic acid anhydride acetic anhydride is used.

3. Process according to patent claims 1 and 2, characterized in that as tertiary amine triethylamine is used.

4. Process according to patent claims 1 to 3, characterized in that as carboxylic acid nitrile aliphatic carboxylic acid nitriles having 1 to 8 carbon atoms in the carboxylic acid résidue or aromatic carboxylic acid nitriles are used.

5. Process according to patent claims 1 to 4, characterized in that per mole of N-oxide 1 to 5 moles of anhydride, 2 to 5 moles of tertiary amine 15 to 80 moles of nitrile are used.

6. Process according to patent claims 1 to 5, characterized in that the reaction is carried out at a temperature of 0 to 90 °C, preferably at 20 to 80 °C.

**Revendications**

1. Procédé pour la préparation de 2-aminopyridines de formule générale

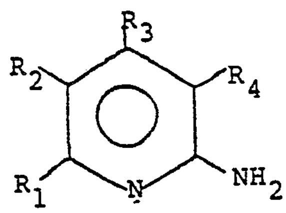

ainsi que de 2-aminoquinoléine à partir de N-oxydes d'acide pyridine-2-carboxylique de formule générale

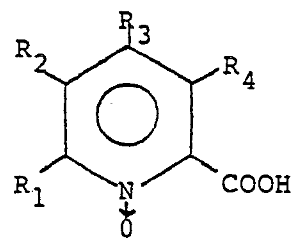

dans laquelle $R_1$, $R_2$, $R_3$ et $R_4$ sont identiques ou différents et représentent H, un groupe —COOH, un groupe alcoyle en $C_1$ à $C_8$ ou un groupe aryle, ou à partir de N-oxyde d'acide quinaldique, caractérisé en ce que l'on fait réagir les N-oxydes cités avec des anhydrides d'acides carboxyliques aliphatiques ayant jusqu'à 6 atomes de C et avec une amine tertiaire en présence de nitriles d'acide carboxylique et que l'on hydrolyse le produit réactionnel.

2. Procédé selon la revendication 1, caractérisé en ce que l'on utilise l'anhydride acétique en tant qu'anhydride d'acide carboxylique.

3. Procédé selon les revendications 1 et 2, caractérisé en ce que l'on utilise la triéthylamine en tant qu'amine tertiaire.

4. Procédé selon les revendications 1 à 3, caractérisé en ce que l'on utilise en tant que nitrile d'acide carboxylique des nitriles d'acide carboxylique aliphatique ayant 1 à 8 atomes de C dans le radical carboxylique ou des nitriles d'acide carboxylique aromatique.

5. Procédé selon les revendications 1 à 4, caractérisé en ce que l'on utilise par mole de N-oxyde 1 à 5 moles d'anhydride, 2 à 5 moles d'amine tertiaire et 15 à 80 moles de nitrile.

6. Procédé selon les revendications 1 à 5, caractérisé en ce que l'on effectue la réaction à une température de 0 à 90 °C, de préférence de 20 à 80 °C.